# EUROPÄISCHE PATENTSCHRIFT

(11) **EP 4 413 337 B1**
(45) Veröffentlichungstag und Bekanntmachung des Hinweises auf die Patenterteilung: **14.01.2026**
(21) Anmeldenummer: 22823035.5
(22) Anmeldetag: 29.11.2022
(51) Int. Cl.: G01G 17/00, G01N 3/62, G01N 33/15

(54) **BESTIMMUNG EINER WIEGE- UND WIEDERHOLGENAUIGKEIT EINER PRÄZISIONSWAAGE**
DETERMINATION OF A WEIGHING AND ITERATION PRECISION OF A PRECISION SCALE
DÉTERMINATION DE LA PRÉCISION D'UNE PESÉE ET DE LA RÉPÉTABILITÉ D'UNE BALANCE DE PRÉCISION

(30) Priorität: 22.12.2021 CH 0707732021
(43) Veröffentlichungstag der Anmeldung: 14.08.2024
(73) Patentinhaber: SOTAX AG, 4147 Aesch (CH)
(72) Erfinder: IMBODEN, Philipp, 3629 Kiesen (CH); BOSS, Thomas, 3645 Gwatt (CH)
(74) Vertreter: Braunpat AG
(86) Internationale Anmeldenummer: PCT/EP2022/083684
(87) Internationale Veröffentlichungsnummer: WO 2023/117335

(56) Entgegenhaltungen:
- CH-A5- 696 371
- DE-B3- 102013 113 126
- DE-U1- 202012 005 821

## Beschreibung

### Technisches Gebiet der Erfindung

Die Erfindung betrifft ein Verfahren und ein System zur Bestimmung einer Wiege- und Wiederholgenauigkeit einer Präzisionswaage, die einen Waagenteller zum Auflegen eines Messobjekts aufweist, sowie eine solche Präzisionswaage und ein Tablettenprüfsystem.

### Stand der Technik

Präzisionswaagen, auch Analysenwaagen oder Mikrowaagen genannt, kommen zum Beispiel in der Chemie- oder Pharmaindustrie oder in Laboren der wissenschaftlichen Forschung zum Einsatz, wenn geringe Massen mit hoher Genauigkeit gemessen werden sollen. Mit derartigen Waagen kann beispielsweise ein Gewicht von 200 Gramm mit einer Auflösung von 0.1 Milligramm gemessen werden. Die Messgenauigkeit ist dabei durch die verwendete Messtechnologie, die Eigenschaften des zu wiegenden Objekts, Verunreinigungen und durch äussere Faktoren am Aufstellort der Präzisionswaage limitiert.

Aus der DE 102 13 786 A1 ist beispielsweise eine Analysenwaage bekannt, die eine motorisch betriebene Windschutztür und eine Sensorik aufweist. Dadurch soll sichergestellt werden, dass der Windschutz nur dann geöffnet wird, wenn eine Probe auf den Waagenteller der Waage aufgebracht wird und der Waagenteller während der Messung von äusseren Einflüssen geschützt bleibt.

Aus DE 20 2012 005 821 U1 ist zudem eine Vorrichtung zum Eichen und Prüfen einer Testvorrichtung für die Härte oder Bruchfestigkeit von Tabletten in einem Tablettenprüfsystem bekannt, welche Testvorrichtung mit einer Kraftmessvorrichtung ausgestattet ist und wobei ein an einem Hebe angebrachtes Referenzgewicht bei Gebrauch der Vorrichtung auf die Kraftmessvorrichtung wirkt und diese mit einer Kraft beaufschlagt.

Bei der industriellen Produktion von pharmazeutischen oder chemischen Erzeugnissen, wie etwa Tabletten, Pillen, Oblongs oder Pellets bestehen oft erschwerte äussere Bedingungen für eine zuverlässig Durchführung von präzisen Messungen. Beispielweise wird die Messung durch Luftzug, Druckschwankungen und Vibrationen verfälscht. Die Waagen müssen daher regelmässig einer Überprüfung und Kalibrierung unterzogen werden. Gleichzeit sind die Präzisionswaagen oftmals in eine Fertigungsanlage, insbesondere in Produktprüfsysteme, integriert und schwer zugänglich.

Eine Prüfung und Kalibrierung der Präzisionswaagen erfolgt anhand der Bestimmung der Wiederholbarkeit und Genauigkeit der Messungen. Weiter wird eine Mindesteinwaage bestimmt, welche die untere Messgrenze angibt, an der die Waage noch ausreichend genau misst. Die Anforderungen werden zum Beispiel durch standardisierte Gewichtstoleranzen bestimmt, die für ein Produkt oder ein Analyseverfahren erlaubt sind. Diese Faktoren werden in der Regel vom Hersteller der Präzisionswaage oder einem zertifizierten Prüfer am Aufstellort der Waage bestimmt und zertifiziert.

Eine Kalibrierung erfolgt in der Regel durch mehrmaliges, d.h. wiederholtes, Auflegen durch einen zertifizierten Prüfer eines externen Referenzgewichts auf den Waagenteller und durch Erfassen von Wiegemesswerten zu dem Referenzgewicht. Diese Werte werden zur Berechnung der obigen Parameter verwendet. Nachteilig an diesem Verfahren ist, dass das Auflegen und Abnehmen des Referenzgewichts von Hand erfolgt und dabei die Messung verfälschen kann. Die Referenzgewichte für Präzisionswaagen sind zudem sehr klein, wodurch deren Handhabung erschwert ist. Ferner ist dieses Vorgehen ungünstig, falls die Präzisionswaage zum Beispiel in einer industriellen Umgebung, wie etwa in einem Produktionsraum mit Unterdruckschwankungen, Luftzug, usw. betrieben wird. Die berechnete Mindesteinwaage, Wiederholbarkeit und Genauigkeit werden durch diese Einflüsse in der Tendenz schlechter oder es gibt im schlimmsten Fall gar keine stabilen Messwerte.

Es ist daher eine Aufgabe der vorliegenden Erfindung, ein Verfahren und ein System zur Bestimmung einer Wiege- und Wiederholgenauigkeit einer Präzisionswaage zu schaffen, welche die oben genannten Nachteile vermeiden, insbesondere soll eine zuverlässige und reproduzierbare Kalibrierung der Präzisionswaage auch bei erschwerten Umgebungseinflüssen und erschwertem Zugang zur Präzisionswaage ermöglicht werden.

### Zusammenfassung der Erfindung

Diese Aufgabe wird erfindungsgemäss durch ein Verfahren zur Bestimmung einer Wiege- und Wiederholgenauigkeit einer Präzisionswaage nach Anspruch 1, ein System zur Bestimmung einer Wiege- und Wiederholgenauigkeit einer Präzisionswaage nach Anspruch 8, eine Präzisionswaage nach Anspruch 20 und ein Tablettenprüfsystem nach Anspruch 22 gelöst. Vorteilhafte Ausgestaltungen und unterschiedliche Ausführungsvarianten der Erfindung gehen aus den abhängigen Ansprüchen hervor.

Ein Verfahren nach der vorliegenden Erfindung zur Bestimmung einer Wiege- und Wiederholgenauigkeit einer Präzisionswaage, insbesondere einer Präzisionswaage in einem Tablettenprüfsystem, die einen Waagenteller zum Auflegen eines Messobjekts aufweist, umfasst folgende Schritte.

Es wird eine Positioniervorrichtung zum automatisierten Positionieren eines Referenzgewichts auf dem Waagenteller, eine Antriebseinheit zum beweglichen Antreiben der Positioniervorrichtung relative zum Waagenteller und eine Bestimmungseinheit bereitgestellt. Die Positioniervorrichtung kann zum Beispiel auch eine Steuereinheit und eine Sensorik umfassen, welche die Positionierung sensorisch erfassen und die Positioniervorrichtung derart steuern, dass das Referenzgewichts auf dem Waagenteller platziert oder davon entfernt wird.

Es wird ein Tariermesswerts erfasst, während der Waagenteller leer ist, um die Präzisionswaage zu tarieren. Demnach erfolgt eine Gewichtsmessung, bei welcher der Waagenteller frei von Objekten ist und lediglich ein system-bedingtes Eigengewicht der Präzisionswaage und andere Einflüsse, die auf den Waagenteller einwirken, erfasst werden. Der Tariermesswert kann zum Abnullen der Präzisionswaage dienen, wie es bei Kalibrierungsverfahren üblich ist.

Anschliessend erfolgt ein automatisiertes Positionieren eines Referenzgewichts auf dem Waagenteller mittels der Positioniervorrichtung. Ein manueller Eingriff zur Handhabung des Referenzgewichts ist nicht erforderlich. Die Masse des Referenzgewichts kann zum Beispiel entsprechend einem bevorzugten Messbereich ausgewählt werden. Beispielsweise entsprechend einem Mittelwert einer zu messenden Einwaage. Die Positionierung erfolgt vorteilhaft zumindest annährend mittig auf dem Waagenteller.

Nach dem Positionieren des Referenzgewichts wird ein erster Wiegemesswerts für das Referenzgewicht mittels der Bestimmungseinheit erfasst.

Anschliessend erfolgt ein automatisiertes Abnehmen des Referenzgewichts von dem Waagenteller und ein erneutes Positionieren desselben Referenzgewichts auf dem Waagenteller mittels der Positioniervorrichtung. Optional kann nach dem Abnehmen des Referenzgewichts und vor dem erneuten Positionieren ein weiterer Tariermesswert erfasst werden, der zur Überprüfung einer Nullposition (Ein- und Auswaage) der Präzisionswaage nach einer Wiegemessung dienen kann.

Nach dem erneuten Positionieren des Referenzgewichts wird ein zweiter Wiegemesswerts für das Referenzgewicht mittels der Bestimmungseinheit erfasst.

Letztlich wird die Wiegegenauigkeit der Präzisionswaage durch die Bestimmungseinheit aus den erfassten Wiegemesswerten bestimmt. Hierfür können herkömmliche statistische Verfahren verwendet werden, wie sie zur Bestimmung einer Wiegegenauigkeit bei Präzisionswaagen bekannt sind. Insbesondere werden bei derartigen Verfahren aus der Gesamtheit der Wiegemesswerte ein Mittelwert der Masse des Messobjekts, eine Standardabweichung, eine Verteilung der Messwerte und der gleichen statistische Parameter bestimmt.

Bevorzugt werden bei dem Verfahren nach der Erfindung mehrere Wiegemesswert erfasst, indem das automatisierte Abnehmen und erneute Positionieren desselben Referenzgewichts auf dem Waagenteller wiederholt und jeweils ein zugehöriger Wiegemesswert für das Referenzgewicht erfasst wird. Vorteilhaft werden 5 - 20 Wiegemesswerte erfasst, besonders vorteilhaft 10 Wiegemesswert, um eine ausreichende Datenmenge für eine zuverlässige Bestimmung der Wiege- und Wiederholgenauigkeit zu haben und gleichzeitig eine effiziente Durchführung der Bestimmung zu ermöglichen. Die Anzahl der für die Genauigkeitsbestimmung zu erfassenden Wiegemesswert kann auch statistisch durch die Bestimmungseinheit determiniert werden. Beispielsweise kann die Anzahl der Wiederholungen der Referenzmessung von einem vorbestimmten Konfidenzintervall bestimmt werden. Die Referenzmessung wird so oft wiederholt, bis die Anforderungen gemäss des vorbestimmten Konfidenzintervalls erfüllt sind.

Als eine automatisierte Positionierung bzw. eine automatisierte Bewegung wird dabei eine maschinelle und elektronisch gesteuerte Handhabung des Referenzgewichts beim Platzieren und Abnehmen des Gewichts auf dem Waagenteller durch die Positioniervorrichtung verstanden. Dabei wird die Positioniervorrichtung durch die Antriebseinheit angetrieben.

Das Verfahren zur Bestimmung einer Wiege- und Wiederholgenauigkeit einer Präzisionswaage nach der vorliegenden Erfindung erfolgt somit ohne Eingriff von aussen innerhalb eines geschlossenen Systems. Insbesondere ist bei dem Verfahren kein Benutzereingriff erforderlich. Äussere Einflüsse während der Messung einer Mindesteinwaage, einer Wiege- und Wiederholgenauigkeit auf das System sind daher weitgehend ausgeschlossen. Die Bestimmung der Wiegegenauigkeit der Präzisionswaage kann direkt am Aufstellort unter den vorliegenden Umgebungsbedingungen erfolgen. Insbesondere kann die Bestimmung auch in eingebautem Zustand der Präzisionswaage durchgeführt werden. Dadurch wird die Bestimmung der Wiege- und Wiederholgenauigkeit effizienter und zuverlässiger als bei herkömmlichen Verfahren.

Bei einer bevorzugten Ergänzung des Verfahrens nach der vorliegenden Erfindung wird spätestens vor dem Erfassen eines Tariermesswerts eine Abdeckung über dem Waagenteller und der Positioniervorrichtung angeordnet, die den Waagenteller vor Umgebungseinflüssen schützt. Die Abdeckung dient dazu Umgebungseinflüsse von dem Waagenteller und der Positioniervorrichtung fern zu halten und insbesondere kurzfristige Beeinträchtigungen abzuwehren.

In einer vorteilhaften Ausgestaltung des Verfahrens nach der vorliegenden Erfindung erfolgt das automatisierte Positionieren und/oder das automatisierte Abnehmen des Referenzgewicht durch eine zumindest überwiegend vertikale und/oder durch eine zumindest überwiegend horizontale Bewegung der Positioniervorrichtung. Demnach wird das Referenzgewicht im Wesentlichen entweder von oberhalb des Waagentellers durch eine Auf-/Abbewegung auf dem Waagenteller platziert und abgenommen oder durch eine im Wesentlichen seitliche, also horizontale Schiebebewegung auf den Waagenteller und von dem Waagenteller wegbewegt. Dadurch können die zurückzulegenden Wege des Referenzgewichts im Vergleich zu Bewegungen in mehreren Richtungen vereinfacht werden.

Bei einer weiteren vorteilhaften Ergänzung des Verfahrens nach der vorliegenden Erfindung wird der Waagenteller durch eine von der Antriebseinheit angetriebene Reinigungseinheit vor dem Erfassen des Tariermesswerts und/oder vor dem Positionieren des Referenzgewichts gereinigt. Als Reinigungseinheit kann beispielsweise eine Bürste, ein Pinsel oder ein Abstreifer verwendet werden. Es kann auch ein Druckluftstoss verwendet werden. Das Reinigen des Waagentellers beseitigt Verunreinigungen auf dem Teller, welche das Wiegen und damit die Bestimmung der Wiege- und Wiederholgenauigkeit beeinträchtigen können.

Vorteilhaft kann das erfindungsgemässe Verfahren mittels einem System zur Bestimmung einer Wiege- und Wiederholgenauigkeit einer Präzisionswaage, insbesondere einer Präzisionswaage in einem Tablettenprüfsystem, wie nachfolgend beschrieben ausgeführt werden.

Nach der vorliegenden Erfindung umfasst ein System zur Bestimmung einer Wiege- und Wiederholgenauigkeit einer Präzisionswaage, insbesondere einer Präzisionswaage in einem Tablettenprüfsystem, die einen Waagenteller zum Auflegen eines Messobjekts aufweist, eine Positioniervorrichtung zum automatisierten Positionieren eines Referenzgewichts auf dem Waagenteller, eine Antriebseinheit zum Antreiben der Positioniervorrichtung relative zum Waagenteller und eine Bestimmungseinheit, die zum Erfassen von Wiegemesswerten an den Waagenteller gekoppelt ist und die Wiege- und Wiederholgenauigkeit der Präzisionswaage bestimmt.

Die Positioniervorrichtung ist dabei zum Beispiel mit einem Mitnehmer derart ausgebildet, dass sie das Referenzgewicht bei einer automatisierten Bewegung des Mitnehmers mitbewegt. Als Mitnehmer kann beispielsweise ein Greifer, ein Schieber oder dergleichen vorgesehen sein, durch deren Bewegung das Referenzgewicht auf dem Waagenteller positioniert und wieder davon entfernt werden kann. Der Mitnehmer wird durch die Antriebseinheit maschinell angetrieben. Durch eine elektronische Steuerung wird das Referenzgewicht auf den Waagenteller aufgebracht und wieder davon entfernt. Die Steuerung kann dabei zum Beispiel durch ein Steuermodul in der Antriebseinheit, in der Bestimmungseinheit oder in der Positioniervorrichtung selbst erfolgen. Es können auch bestehende Steuereinrichtungen einer Produktionsanlage, der Präzisionswaage, oder eines Prüfsystems, insbesondere eines Tablettenprüfsystems, zum Prüfen des Messobjekts zur Steuerung der Positioniervorrichtung verwendet werden. Das Zuführen des Referenzgewichts in das System, insbesondere zu der Positioniervorrichtung, kann dabei ebenfalls automatisiert aber auch manuell erfolgen.

Wie oben erwähnt, kann die Bestimmung der Wiege- und Wiederholgenauigkeit einer Präzisionswaage mit einem solchen System ohne Eingriff von aussen erfolgen. Die Messung der Wiegemesswerte wird von äusseren Einflüssen nicht beeinträchtigt. Die Bestimmung der Wiegegenauigkeit der Präzisionswaage kann direkt am Einsatzort erfolgen, auch während die Präzisionswaage in ein Prüfsystem, insbesondere in ein Tablettenprüfsystem, eingebettet ist.

Vorteilhaft umfasst das System eine von der Antriebseinheit angetriebene Reinigungseinheit zur Reinigung des Waagentellers. Beispielsweise kann eine Bürste, ein Pinsel oder ein Abstreifer mittels einer horizontalen Bewegung über den Waagenteller geführt werden, so dass Verunreinigungen weggewischt werden. Der Reinigungsvorgang kann somit ebenfalls automatisiert erfolgen, wodurch die Genauigkeit der Bestimmung der Wiegemesswerte erhöht wird.

In einer Ausgestaltung des erfindungsgemässen Systems ist eine Abdeckung vorgesehen, die beim Erfassen von Wiegemesswerten abnehmbar über dem Waagenteller und der Positioniervorrichtung angeordnet ist und den Waagenteller vor Umgebungseinflüssen schützt. Zum Einbringen eines Referenzgewichts in das System kann die Abdeckung abgenommen werden, so dass die Positioniervorrichtung zur Aufnahme des Referenzgewicht zugänglich ist. Die Abdeckung reduziert Beeinträchtigungen der Messwertbestimmung, wie oben erwähnt.

In einer vorteilhaften Variante des Systems zur Bestimmung einer Wiege- und Wiederholgenauigkeit einer Präzisionswaage nach der vorliegenden Erfindung sind die Positioniervorrichtung, die Antriebseinheit und/oder die Bestimmungseinheit durch Baumodule der Präzisionswaage oder des Tablettenprüfsystems gegeben und/oder mit Baumodulen der Präzisionswaage oder des Tablettenprüfsystems gekoppelt. Beispielsweise kann ein Antriebsmodul der Präzisionswaage oder des Tablettenprüfsystems als Antriebseinheit für die Positioniervorrichtung dienen. Ein Steuermodul der Präzisionswaage oder des Tablettenprüfsystems kann die Positionierung des Referenzgewichts durch die Positioniervorrichtung steuern. Ein Wiegemodul der Präzisionswaage oder des Tablettenprüfsystems kann zum Erfassen der Wiegemesswerte dienen und die Messwerte an die Bestimmungseinheit liefern.

Besonders vorteilhaft ist das erfindungsgemässe System in ein Prüfsystem, wie etwa ein Tablettenprüfsystem, zur Qualitätsprüfung des Wiegeobjekts integriert. Das Prüfsystem kann wiederum in eine Produktionsanlage zur Produktion des Wiegeobjekts integriert sein. Beispielsweise ist das Wiegeobjekt ein pharmazeutisches Produkt, wie eine Tablette, Pille, Pellet oder dergleichen. Nach der Herstellung werden diese dem Prüfsystem zum Beispiel für eine computergesteuerte Vermessung von Gewicht, Dicke, Durchmesser und Bruchfestigkeit zugeführt. Die automatisierte Bestimmung der Wiege- und Wiederholgenauigkeit der Präzisionswaage nach der vorliegenden Erfindung ist ein Wesentlicher Bestandteil eines Tablettenprüfsystem in der Qualitätskontrolle.

In einer Ausführungsvariante des Systems zur Bestimmung einer Wiege- und Wiederholgenauigkeit einer Präzisionswaage nach der vorliegenden Erfindung ist die Positioniervorrichtung als zumindest überwiegend vertikal beweglicher Greifer zum automatisierten Ergreifen und Freigeben des Referenzgewichts ausgebildet. Der Greifer kann als Greifarm ausgebildet sein. Weiter kann der Greifer zwei zueinander bewegliche Klemmstücke zum Einklemmen des Referenzgewichts aufweisen. Der Greifer wird vorteilhaft von einer Steuereinrichtung automatisch gesteuert, um den Greifer über dem Waagenteller anzuordnen und vertikal auf und ab zu bewegen. Eine Sensorik der Steuereinrichtung kann dabei die Positionierung des Greifers bestimmen, sodass ein Referenzgewicht im Wesentlichen mittig auf dem Waagenteller abgesetzt und von diesem abgenommen werden kann.

In einer anderen Ausführungsvariante des Systems zur Bestimmung einer Wiege- und Wiederholgenauigkeit einer Präzisionswaage nach der vorliegenden Erfindung ist die Positioniervorrichtung als horizontal bewegliche Aufnahme zum automatisierten Verschieben des Referenzgewichts ausgebildet. Das Referenzgewicht kann dabei in der Aufnahme aufgenommen sein und gemeinsam mit dieser seitlich verschoben werden. Eine Steuereinrichtung kann dabei die Antriebseinheit steuern, welche die Aufnahme antreibt. Somit kann die Aufnahme mittels der Antriebseinheit automatisch über dem Waagenteller positioniert werden, so dass das Referenzgewicht auf dem Waagenteller zu liegen kommt. Eine horizontale Bewegung zur Positionierung des Referenzgewichts kann den Bauraum in der Präzisionswaage, bzw. dem System zur Bestimmung der Wiege- und Wiederholgenauigkeit der Präzisionswaage, effizient nutzen.

Bei einer vorteilhaften Ausführungsform dieser Ausführungsvariante des Systems kann die Aufnahme hülsenartig zum losen Aufnehmen des Referenzgewichts ausgebildet sein. Als hülsenartige Aufnahme ist eine Aufnahme zu verstehen, die an gegenüberliegenden Seiten, etwa oben und unten, offen ist und vorzugsweise einen geschossenen Umfang aufweist. Bei dem losen Aufnehmen des Referenzgewichts ist das Referenzgewicht nicht mit der Aufnahme verbunden, sondern ist frei beweglich innerhalb des Umfangs der Aufnahme vorgesehen. Ein Umfang der Aufnahmen ist beispielsweise kreisförmig. Er könnte aber auch oval, elliptisch, rautenartig oder dergleichen sein. Vorteilhaft weist die Aufnahme einen runden Durchmesser auf. Bei einem seitlichen Bewegen der Aufnahme wird das Referenzgewicht durch die runde Form einer Innenseite des Umfangs automatisch innerhalb der Aufnahme positioniert.

Weiter kann eine ebene Plattform vorgesehen sein, die mit dem Waagenteller eine Ebene bildet und den Waagenteller zumindest teilweise einschliesst. Die Aufnahme kann zum Verschieben des Referenzgewichts derart horizontal beweglich angeordnet sein, dass sie mittels der Antriebseinheit der Plattform über den Waagenteller und von dem Waagenteller über die Plattform beweglich ist. Dadurch kann das Referenzgewicht auf den Waagenteller geschoben und auch wieder von diesem weggeschoben werden. Es erfolgt also eine Positionierung des Referenzgewichts im Sinne der Erfindung. Vorzugsweise wird die Aufnahme nach dem Aufschieben des Referenzgewicht geringfügig entgegen der Aufschieberichtung verschoben, sodass der Umfang der Aufnahme von dem Referenzgewicht beabstandet wird und das Referenzgewicht frei auf dem Waagenteller liegt. Die Bestimmungseinheit ist zum Erfassen der Wiegemesswerte an den Waagenteller gekoppelt und bestimmt daraus die Wiegegenauigkeit der Präzisionswaage.

Vorteilhaft kann eine zweite Aufnahme vorgesehen sein, die ebenso wie die erste Aufnahme derart an der Antriebseinheit angeordnet ist, dass sie von der Plattform über den Waagenteller bewegbar ist. Die zweite Aufnahme ist vorzugsweise identisch mit der ersten Aufnahme für das Referenzgewicht. Mit der zweiten Aufnahme kann eine Messumgebung über dem Waagenteller simuliert werden, die im Wesentlichen die gleiche Umgebung darstellt, wie bei einer Messung des Referenzgewichts. Bei einer Tariermessung liegen somit die identischen Bedingungen vor, wie bei einer Messung des Referenzgewichts, ausser dass, das Referenzgewicht in der Aufnahme vorhanden ist. Dadurch entspricht die Tariermessung möglichst genau einer Messung des Referenzgewichts.

Bei einer weiteren vorteilhaften Ausführungsform dieser Ausführungsvariante des Systems kann die Antriebseinheit eine Drehsachse mit wenigstens einem davon abstehenden Dreharm aufweisen. Dabei ist die Aufnahme derart am Dreharm angeordnet, dass die Aufnahme um die Drehachse beweglich ist. Somit kann die Aufnahme eine Schwenk- bzw. Kreisbewegung durchführen, die sie über den Waagenteller bewegt und davon entfernt. Bei einer fortlaufenden Rotation des Dreharms kann die Aufnahme auch wiederkehrend über dem Waagenteller zu liegen kommen. Die Antriebseinheit ist dabei als Rotationseinheit ausgebildet, welche zum Beispiel durch ein Modul des Prüfsystems, wie etwa einem Tablettenprüfsystem, gegeben sein kann, wie oben beschrieben.

Bei dieser Ausführungsvariante des Systems nach der Erfindung kann zum Beispiel ein Drehstern mit wenigstens zwei Dreharmen vorgesehen sein, der mit der Drehachse der Antriebseinheit koppelbar ist. Die Aufnahme für das Referenzgewicht kann an einem Dreharm angeordnet sein und die Reinigungseinheit und/oder eine zweite Aufnahme können jeweils an einem weiteren Dreharm angeordnet sein. Somit sind die Aufnahme für das Referenzgewicht und die Reinigungseinheit und/oder die zweite Aufnahme gemeinsam um die Drehachse über der Plattform beweglich. Vorzugsweise sind drei Dreharme vorgesehen, je einer für die Aufnahme des Referenzgewichts, für eine leere bleibende Aufnahme und für die Reinigungseinheit.

Vorteilhaft schliesst bei dieser Ausführungsform die Plattform den Waagenteller wenigstens an gegenüberliegenden Seiten ein. Besonders bevorzugt wird der Waagenteller vollständig umschlossen, so dass der Waagenteller plan mit der Ebene der Plattform abschliesst. Hierfür kann die Plattform zum Beispiel höhenverstellbar auf einer den Waagenteller tragenden Fläche eines Tablettenprüfsystems aufsetzbar angeordnet sein. Dadurch kann die Höhe der Plattform an die Ebene des Waagentellers angepasst werden. Alternativ kann auch der Waagenteller höhenverstellbar an der Präzisionswaage gelagert sein. Dadurch kann der Waagenteller an eine Position der Plattform angepasst werden.

Nach einem weiteren Aspekt der Erfindung ist eine Präzisionswaage mit einem Waagenteller zum Auflegen eines Messobjekts vorgesehen, die ein System zur Bestimmung einer Wiege- und Wiederholgenauigkeit wie vorher beschrieben umfasst. Vorteilhaft umfasst die Präzisionswaage ein Antriebsmodul, ein Steuermodul und ein Wiegemodul. Das Antriebsmodul und das Steuermodul dienen als Antriebseinheit und als Steuereinheit für eine Positioniervorrichtung des Systems. Das Wiegemodul dient zum Erfassen von Wiegewerten für die Bestimmungseinheit des Systems, wie oben beschrieben.

Die erfindungsgemässe Präzisionswaage kann zeitsparend kalibriert werden. Durch die wiederholte automatisierte Positionierung eines Referenzgewichts kann eine Reihe von Wiegemesswerten des Referenzgewichts schnell und reproduzierbar erfasst werden. Aus diesen Messwerten können die Wiege- und Wiederholgenauigkeit und die Mindesteinwaage für die Präzisionswaage zuverlässig berechnet werden. Eine manuelle Handhabung des Referenzgewichts ist während dem gesamten Kalibriervorgang nicht erforderlich.

Die Ziele der Erfindung werden auch durch ein Tablettenprüfsystem umfassend einer Präzisionswaage gemäss vorliegender Erfindung erreicht.

### Kurzbeschreibung der Zeichnungen

Eine Ausführungsvariante der Erfindung wird im Folgenden anhand der Figuren dargestellt, die lediglich zur Erläuterung dienen und nicht einschränkend auszulegen sind. Aus den Figuren offenbar werdende Merkmale der Erfindung sollen einzeln und in jeder Kombination als zur Offenbarung der Erfindung gehörend betrachtet werden. In den Zeichnungen zeigen:
Fig. 1: eine schematische Darstellung einer Anordnung von Bauteilen eines Systems zur Bestimmung einer Wiege- und Wiederholgenauigkeit einer Präzisionswaage auf einer Oberfläche der Präzisionswaage oder des Tablettenprüfsystems nach der Erfindung;
Fig. 2: eine schematische Darstellung eines Systems zur Bestimmung einer Wiege- und Wiederholgenauigkeit analog Figur 1;
Fig. 3: eine schematische Darstellung einer Reinigung eines Waagentellers mit dem System aus Figur 2 bei einem Verfahren zur Bestimmung einer Wiege- und Wiederholgenauigkeit der Präzisionswaage nach der Erfindung;
Fig. 4: eine schematische Darstellung des Systems aus Figur 2 mit einem Referenzgewicht;
Fig. 5: eine schematische Darstellung des Systems aus Figur 2 beim Erfassen eines Tariermesswerts gemäss dem erfindungsgemässen Verfahren;
Fig. 6a: eine schematische Darstellung des Systems aus Figur 2 in einer ersten Position zum Positionieren des Referenzgewichts auf dem Waagenteller;
Fig. 6b: eine schematische Darstellung des Systems aus Figur 2 in einer zweiten Position zum Positionieren des Referenzgewichts auf dem Waagenteller; und
Fig. 7: eine schematische Darstellung des Systems aus Figur 2 beim Erfassen eines Wiegemesswerts gemäss dem erfindungsgemässen Verfahren.

### Bevorzugte Ausführungsformen der Erfindung

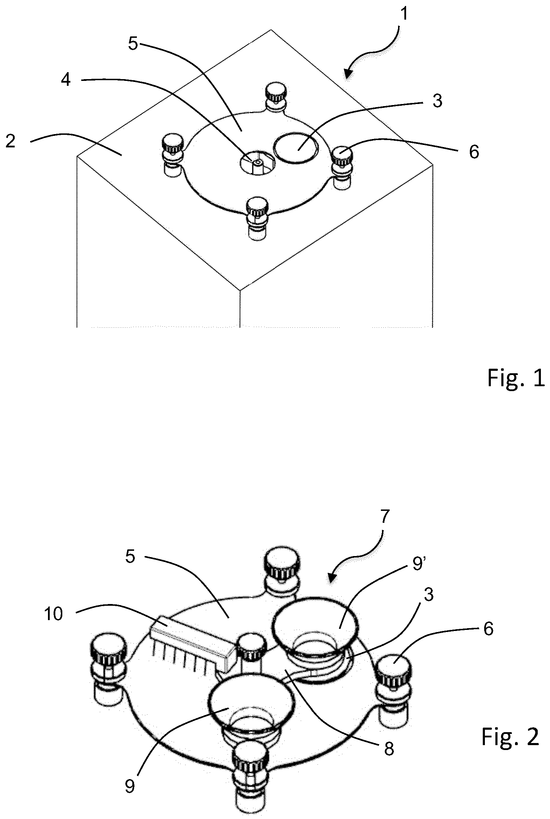

In den Figuren 1 bis 7 ist eine vereinfachte schematische Darstellung einer Ausführungsvariante eines Systems zur Bestimmung einer Wiege- und Wiederholgenauigkeit einer Präzisionswaage nach der Erfindung gezeigt, um die allgemeinen Merkmale der Erfindung anhand dieser Ausführungsvariante vorzustellen und Details zu dieser Ausführungsvariante zu erläutern. Die in den Figuren gezeigte Ausführungsvariante bezieht sich auf ein System, bei dem das automatisierte Positionieren und/oder das automatisierte Abnehmen des Referenzgewicht durch eine zumindest überwiegend horizontale Bewegung der Positioniervorrichtung erfolgt, wie unten genauer ausgeführt wird. Es wird jedoch betont, dass die eingangs beschriebene Ausführungsvariante eines Systems zur Bestimmung einer Wiege- und Wiederholgenauigkeit einer Präzisionswaage mit einem automatisierten Positionieren und/oder Abnehmen des Referenzgewicht durch eine zumindest überwiegend vertikale Bewegung der Positioniervorrichtung ebenfalls zum Umfang der vorliegenden Erfindung gehört, obwohl hierzu keine Beschreibung anhand von Figuren erfolgt.

In Figur 1 ist eine Präzisionswaage 1, zum Beispiel eines Tablettenprüfsystems, schematisch als Block dargestellt. Die Präzisionswaage 1 kann Bestandteile eines Prüfsystems zur Überprüfung von Wiegeobjekten sein, insbesondere eines Tablettenprüfsystems zur Prüfung von Tabletten, Pillen, Oblongs, Pellets oder dergleichen. Die Präzisionswaage oder das Tablettenprüfsystem weist eine Oberfläche 2 auf, die einen Waagenteller 3 zum Wiegen von Wiegeobjekten (nicht gezeigt) trägt. Der Waagenteller 3 und die Oberfläche 2 sind im Wesentlichen horizontal ausgerichtet, so dass die Wiegeobjekte frei darauf angeordnet werden können, ohne davon abzurutschen oder abzurollen. Durch die Oberfläche 2 ist eine Antriebsachse 4 zugänglich, die von einer Antriebseinheit rotierend angetrieben wird.

Auf der Oberfläche 2 ist eine ebene Plattform 5 des Systems zur Bestimmung einer Wiege- und Wiederholgenauigkeit der Präzisionswaage aufgesetzt. Die Plattform 5 ist mittels vier Rändelschrauben 6 relative zur Oberfläche 2 und zum Waagenteller 3 höhenverstellbar. Die Höhe der Plattform ist derart eingestellt, dass die Oberseite der Plattform 5 mit dem Waagenteller eine horizontale Ebene bildet. Dabei ist der Waagenteller 3 in einer Aussparung in der Plattform 5 angeordnet, so dass die Plattform den Waagenteller umschliesst. In der Mitte der Plattform 5 ist eine Öffnung durch die Plattform vorgesehen, durch welche die Antriebsachse 4 zugänglich ist. Es ist auch möglich, dass der Waagenteller 3 in der Präzisionswaage höhenverstellbar gelagert ist. So kann eine Feinjustierung der horizontalen Ebene erfolgen.

Wie in Figur 2 ersichtlich, ist auf der Plattform 5 eine Positioniervorrichtung 7 des Systems zur Bestimmung der Wiege- und Wiederholgenauigkeit einer Präzisionswaage aufgesetzt. Die Positioniervorrichtung 7 umfasst einen Drehstern mit drei Dreharmen 8, die im gleichen Winkel um eine Rotationsachse des Drehsterns angeordnet sind. Der Drehstern ist an einem Ende der Antriebsachse gekoppelt und kann von der Antriebseinheit automatisiert angetrieben werden. Die Antriebseinheit ist hierfür an eine Steuerung angeschlossen, die in dieser Ausführungsvariante von einem Steuermodul eines Tablettenprüfsystems bereitgestellt wird.

An zwei der Dreharmen 8 ist jeweils eine hülsenartige Aufnahme 9 und 9' vorgesehen. An dem dritten Dreharm ist eine Reinigungseinheit 10 in Form einer Bürste angeordnet. Die Aufnahmen 9 und 9' und die Bürste sind so weit von der Rotationsachse des Drehsterns beabstandet angeordnet, dass sie bei einer Rotation um die Drehachse über den Waagenteller 3 bewegt werden. Vorteilhaft sind die Aufnahmen 9 und 9' derart an den Dreharmen 8 befestigt, dass sie den Waagenteller 3 nicht berühren.

Die Aufnahmen 9 und 9' sind in dieser Ausführungsvariante identisch ausgebildet. Sie sind nach oben und nach unten offen. Ein Umfang der Aufnahmen ist bei dieser Ausführungsform vorzugsweise kreisförmig. Er könnte aber auch oval, elliptisch, rautenartig oder dergleichen sein. Die Aufnahmen sind daher nach der Art einer Hülse mit einem Durchgang und einer Umfangswand ausgebildet. An der oberen Seite der Aufnahme, die von der Plattform 5 abgewandt ist, ist eine trichterförmige Aufweitung des Umfangs vorgesehen. Dadurch kann ein Einbringen eines Referenzgewichts in die Aufnahme erleichtert werden.

Das dargestellte System zur Bestimmung einer Wiege- und Wiederholgenauigkeit einer Präzisionswaage nach der vorliegenden Erfindung umfasst die Positioniervorrichtung 7 mit den Dreharmen 8, die zum automatisierten Positionieren eines Referenzgewichts auf dem Waagenteller 3 vorgesehen ist, die Antriebseinheit mit der Antriebsachse 4 zum Antreiben der Positioniervorrichtung 7 relative zum Waagenteller 3 und eine Bestimmungseinheit (nicht gezeigt), die zum Erfassen von Wiegemesswerten an den Waagenteller 3 gekoppelt ist und die Wiege- und Wiederholgenauigkeit der Präzisionswaage bestimmt. Die Bestimmungseinheit kann zum Beispiel durch ein Wiegemodul der Präzisionswaage gegeben sein oder auch durch ein Computermodul, dem die Wiegemesswerte der Präzisionswaage eingespeist werden. Mittels der Antriebseinheit sind die Dreharme 8 der Positioniervorrichtung 7 als horizontal beweglich und können um die Antriebsachse schwenken.

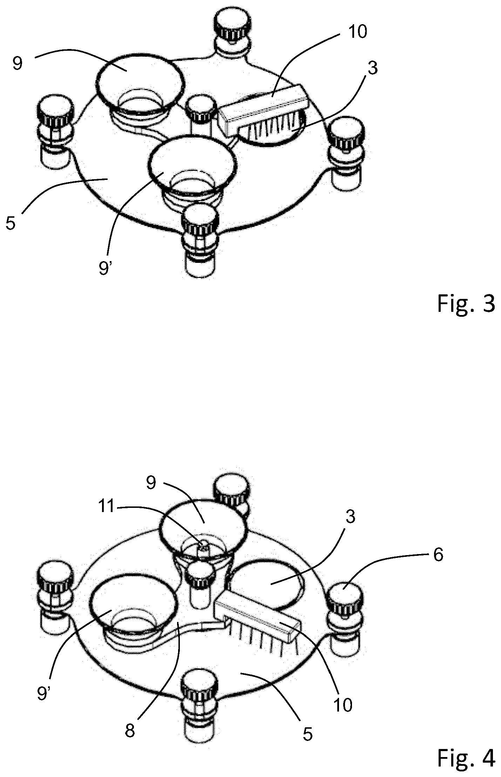

In Figur 3 ist das System bei einer Reinigung des Waagentellers 3 gezeigt. Die Borsten der Bürste sind derart am Dreharm 8 angeordnet, dass sie bei einer Rotation des Drehsterns über die Fläche des Waagentellers 3 streichen und diese reinigen. Dabei kann der Drehstern mehrmals um die Antriebsachse 4 rotieren und somit mehrfach über den Waagenteller 3 streichen. Alternativ kann der Drehstern auch durch Abwechseln der Drehrichtung über dem Waagenteller 3 hin und her bewegt werden. Neben dem Waagenteller 3 kann mittels der Bürste der Reinigungseinheit auch die Plattform 5 gereinigt werden.

Vor oder nach dem Reinigen des Waagentellers 3 kann ein Referenzgewicht 11 in eine der Aufnahmen 9 der Positioniervorrichtung 7 eingebracht werden, wie in Figur 4 dargestellt ist. Die hülsenartige Aufnahme 9 nimmt das Referenzgewicht 11 lose auf. Das heisst das Referenzgewicht 11 ist lose innerhalb des Umfangs der Aufnahme 9 angeordnet und wird von der Aufnahme nicht festgehalten. Das Referenzgewicht 11 kann manuell in die Aufnahme 9 eingelegt oder es kann mit Hilfe einer Zuführvorrichtung in die Aufnahme eingebracht werden. Die Zuführvorrichtung kann zum Beispiel ein Bauelement eines Prüfsystems, wie zum Beispiel einem Tablettenprüfsystem, für zu wiegende Objekte sein, beispielsweise für die Zufuhr von Tabletten zur Präzisionswaage.

Vorzugsweise umfasst das System eine Abdeckung (nicht gezeigt), die abnehmbar über dem Waagenteller 3 und der Positioniervorrichtung 7 angeordnet ist und den Waagenteller 3 vor Umgebungseinflüssen schützt. Die Abdeckung kann zum Beispiel zur Handhabung des Referenzgewichts 11 abgenommen werden.

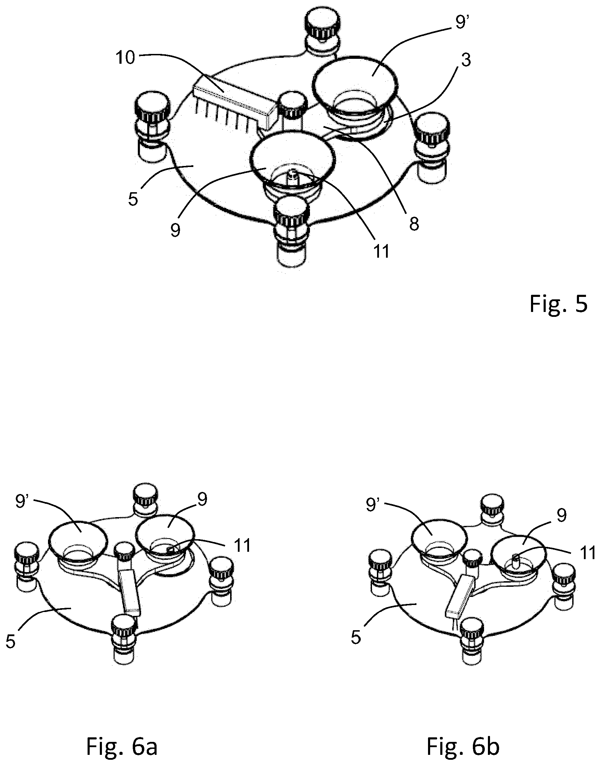

Bei dem Verfahren zur Bestimmung einer Wiege- und Wiederholgenauigkeit der Präzisionswaage nach der vorliegenden Erfindung wird ein Tariermesswert erfasst während der Waagenteller 3 leer ist, um die Präzisionswaage zu tarieren. In Figur 5 ist das System in einer Tarierposition gezeigt, in der die leere Aufnahme 9' durch Drehung der Positioniervorrichtung 7 über dem Waagenteller 3 angeordnet wird. In dieser Position wird der Tariermesswert erfasst. Dabei ist es vorteilhaft, dass die Aufnahmen 9 und 9' identisch sind, somit wird mittels der leeren Aufnahme 9' eine Umgebung über dem Waagenteller 3 simuliert, die auch beim Messen eines Wiegemesswertes vorliegt.

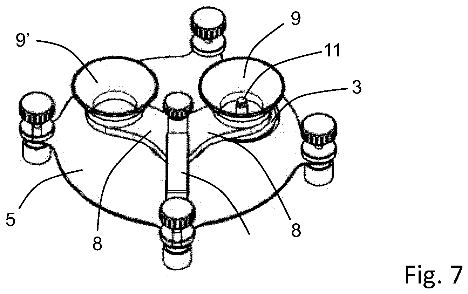

Nach dem Erfassen des Tariermesswerts erfolgt mittels der Positioniervorrichtung 7 ein automatisiertes Positionieren des Referenzgewichts 11, das in der weiteren Aufnahme 9 liegt, auf dem Waagenteller 3. Hierfür wird die Aufnahme 9 mit dem Referenzgewicht 11 mittels der Antriebseinheit automatisiert horizontal verschoben, um das Referenzgewicht 11 auf dem Waagenteller 3 anzuordnen. Vorteilhaft wird dabei eine Hin- und Herbewegung der Aufnahme 9 durchgeführt, bei der die Aufnahme die Bewegungsrichtung wechselt, um das Referenzgewicht 11 mittig auf dem Waagenteller 3 zu positionieren. Wie in Figur 6a gezeigt ist, wird die Aufnahme 9 durch eine Bewegung entgegen dem Uhrzeigersinn auf den Waagenteller geschoben. Dann kehrt die Antriebseinheit die Drehrichtung um und die Aufnahme 9 wird im Uhrzeigersinn zurückgeschoben, bis das Referenzgewicht 11 mittig ausgerichtet ist, wie in Figur 6b dargestellt ist. Zum Erfassen der Zentrierung des Referenzgewichts kann beispielsweise eine Sensorik dienen, die als Modul der Präzisionswaage oder des Tablettenprüfsystems vorgesehen ist. Ist das Referenzgewicht 11 auf dem Waagenteller 3 zentriert, kehrt die Drehrichtung der Aufnahme 9 erneut um und die Aufnahme wird ebenfalls zentriert über dem Waagenteller 3 angeordnet. Da das Referenzgewicht 11 lose in der Aufnahme 9 aufgenommen ist, bleibt es dabei in seiner mittigen Position und liegt beabstandet zum Umfang frei zur Aufnahme 9. Dies entspricht einer Messposition des Systems, wie es in Figur 7 gezeigt ist. Dabei ist die Aufnahme 9 in der gleichen Stellung über dem Waagenteller 3 wie die Aufnahme 9' in der Tarierposition aus Figur 5.

Nun wird ein erster Wiegemesswerts für das Referenzgewicht 11 mittels der Bestimmungseinheit erfasst und in dieser gespeichert.

Anschliessend erfolgt ein automatisiertes Abnehmen des Referenzgewichts 11 von dem Waagenteller 3 durch erneutes Drehen der Aufnahme 9, bis das Referenzgewicht 11 auf der Plattform 5 zu liegen kommt. Danach erfolgt ein erneutes Positionieren desselben Referenzgewichts 11 auf dem Waagenteller 3 in die Messposition durch die Positioniervorrichtung 7, wie vorher beschrieben, und es wird ein zweiter Wiegemesswert für das Referenzgewicht 11 erfasst.

Vorteilhaft wird der Vorgang des Abnehmens und erneuten Positionierens des Referenzgewichts 11 mehrfach wiederholt und es werden mehrere Wiegemesswerte des Referenzgewichts unabhängig voneinander erfasst. In der Regel werden 10 Wiegemesswerte benötigt, um zum Bespiel die Mindesteinwaage nach zurzeit gültigen Normen zu berechnen.

Die Bestimmung der Wiegegenauigkeit der Präzisionswaage aus den erfassten Wiegemesswerten erfolgt durch die Bestimmungseinheit. Die Messwerte können zum Beispiel auf einem Messreport aufbereitet werden, auf dem unter anderem die Mindesteinwaage am Aufstellort, die Wiege- und Wiederholgenauigkeit der Präzisionswaage angegeben werden.

Nach dem Abschluss der Bestimmung der Wiege- und Wiederholgenauigkeit der Präzisionswaage kann das Referenzgewicht 11 manuell oder maschinell von der Plattform 5 entfernt werden. Auch der Drehstern mit den Dreharmen 8 und die Plattform 5 können von der Oberfläche 2 der Präzisionswaage 1 abgenommen werden. Die Präzisionswaage ist nun bereit zur Messung von Wiegeobjekten mit einer vorgegebenen und reproduzierbaren Genauigkeit.

### Bezugszeichenlegende

1 Präzisionswaage
2 Oberfläche
3 Waagenteller
4 Antriebsachse
5 Plattform
6 Rändelschrauben
7 Positioniervorrichtung
8 Dreharme
9, 9'Aufnahme
10Reinigungseinheit
11 Referenzgewicht

## Patentansprüche

1. Verfahren zur Bestimmung einer Wiege- und Wiederholgenauigkeit einer Präzisionswaage (1), insbesondere einer Präzisionswaage in einem Tablettenprüfsystem, die einen Waagenteller (3) zum Auflegen eines Messobjekts aufweist, **dadurch gekennzeichnet, dass** das Verfahren folgende Schritte umfasst:
(a) Bereitstellen einer Positioniervorrichtung (7) zum automatisierten Positionieren eines Referenzgewichts (11) auf dem Waagenteller (3), einer Antriebseinheit zum beweglichen Antreiben der Positioniervorrichtung (7) relativ zum Waagenteller (3) und einer Bestimmungseinheit,
(b) Erfassen eines Tariermesswerts während der Waagenteller (3) leer ist zum Tarieren der Präzisionswaage (1),
(c) automatisiertes Positionieren eines Referenzgewichts (11) auf dem Waagenteller (3) mittels der Positioniervorrichtung (7),
(d) Erfassen eines ersten Wiegemesswerts für das Referenzgewicht (11) mittels der Bestimmungseinheit,
(e) automatisiertes Abnehmen des Referenzgewichts (11) von dem Waagenteller (3) und anschliessendes erneutes Positionieren desselben Referenzgewichts (11) auf dem Waagenteller (3) mittels der Positioniervorrichtung (7),
(f) Erfassen eines zweiten Wiegemesswerts für das Referenzgewicht (11) mittels der Bestimmungseinheit, und
(g) Bestimmung der Wiege- und Wiederholgenauigkeit der Präzisionswaage (1) aus den erfassten Wiegemesswerten durch die Bestimmungseinheit.

2. Verfahren zur Bestimmung einer Wiege- und Wiederholgenauigkeit einer Präzisionswaage nach Anspruch 1, wobei die Schritte (e) und (f) mehrfach wiederholt werden, um mehrere Wiegemesswerte zu erfassen.

3. Verfahren zur Bestimmung einer Wiege- und Wiederholgenauigkeit einer Präzisionswaage nach einem der Ansprüche 1 oder 2, wobei zumindest vor dem Erfassen eines Tariermesswerts eine Abdeckung über dem Waagenteller (3) und der Positioniervorrichtung (7) angeordnet wird, die den Waagenteller (3) vor Umgebungseinflüssen schützt.

4. Verfahren zur Bestimmung einer Wiege- und Wiederholgenauigkeit einer Präzisionswaage nach einem der Ansprüche 1 bis 3, wobei der Waagenteller (3) durch eine von der Antriebseinheit angetriebene Reinigungseinheit (10) vor dem Erfassen des Tariermesswerts und/oder vor dem Positionieren des Referenzgewichts (11) gereinigt wird.

5. System zur Bestimmung einer Wiege- und Wiederholgenauigkeit einer Präzisionswaage (1), insbesondere einer Präzisionswaage in einem Tablettenprüfsystem, die einen Waagenteller (3) zum Auflegen eines Messobjekts aufweist, **dadurch gekennzeichnet, dass** das System umfasst:
- eine Positioniervorrichtung (7) zum automatisierten Positionieren eines Referenzgewichts (11) auf dem Waagenteller (3),
- eine Antriebseinheit zum Antreiben der Positioniervorrichtung (7) relativ zum Waagenteller (3) und
- eine Bestimmungseinheit, die zum Erfassen von Wiegemesswerten an den Waagenteller (3) gekoppelt ist und die Wiege- und Wiederholgenauigkeit der Präzisionswaage (1) bestimmt.

6. System zur Bestimmung einer Wiege- und Wiederholgenauigkeit einer Präzisionswaage nach Anspruch 5, **dadurch gekennzeichnet, dass** eine abnehmbare Abdeckung vorgesehen ist, die zumindest über dem Waagenteller (3) und der Positioniervorrichtung (7) angeordnet ist und den Waagenteller (3) vor Umgebungseinflüssen schützt.

7. System zur Bestimmung einer Wiege- und Wiederholgenauigkeit einer Präzisionswaage nach Anspruch 5 oder 6, **dadurch gekennzeichnet, dass** die Positioniervorrichtung (7), die Antriebseinheit und/oder die Bestimmungseinheit durch Baumodule der Präzisionswaage oder des Tablettenprüfsystems gegeben sind und/oder mit Baumodulen der Präzisionswaage oder des Tablettenprüfsystems gekoppelt sind, beispielsweise einem Antriebmodul, einem Steuermodul und/oder einem Wiegemodul der Präzisionswaage und/oder eines Tablettenprüfsystems.

8. System zur Bestimmung einer Wiege- und Wiederholgenauigkeit einer Präzisionswaage nach einem der Ansprüche 5 bis 7, **dadurch gekennzeichnet, dass** die Positioniervorrichtung (7) als zumindest überwiegend vertikal beweglicher Greifer zum automatisierten Ergreifen und Freigeben des Referenzgewichts (11) ausgebildet ist.

9. System zur Bestimmung einer Wiege- und Wiederholgenauigkeit einer Präzisionswaage nach einem der Ansprüche 5 bis 7, **dadurch gekennzeichnet, dass** die Positioniervorrichtung (7) als horizontal bewegliche Aufnahme (9, 9') zum automatisierten Verschieben des Referenzgewichts (11) ausgebildet ist.

10. System zur Bestimmung einer Wiege- und Wiederholgenauigkeit einer Präzisionswaage nach Anspruch 9, **dadurch gekennzeichnet, dass** die Aufnahme (9, 9') hülsenartig zum losen Aufnehmen eines Referenzgewichts (11) ausgebildet ist und eine ebene Plattform (5) vorgesehen ist, die mit dem Waagenteller (3) eine Ebene bildet und den Waagenteller (3) zumindest teilweise einschliesst, wobei die Aufnahme (9, 9') derart horizontal verschieblich angeordnet ist, dass sie von der Plattform (5) über den Waagenteller (3) und von dem Waagenteller (3) über die Plattform (5) beweglich ist.

11. System zur Bestimmung einer Wiege- und Wiederholgenauigkeit einer Präzisionswaage nach einem der Ansprüche 9 bis 10, **dadurch gekennzeichnet, dass** eine zweite Aufnahme (9') derart an der Antriebseinheit angeordnet ist, dass die zweite Aufnahme (9') von der Plattform (5) über den Waagenteller (3) bewegbar ist, wobei die zweite Aufnahme vorzugsweise identisch ist mit der Aufnahme (9) für das Referenzgewicht (11).

12. System zur Bestimmung einer Wiege- und Wiederholgenauigkeit einer Präzisionswaage nach einem der Ansprüche 9 bis 11, **dadurch gekennzeichnet, dass** die hülsenförmige Aufnahme einen runden Durchmesser aufweist.

13. Präzisionswaage, insbesondere für ein Tablettenprüfsystem, mit einem Waagenteller (3) zum Auflegen eines Messobjekts, die ein System zur Bestimmung einer Wiege- und Wiederholgenauigkeit nach einem der Ansprüche 5 bis 12 umfasst.

14. Präzisionswaage nach Anspruch 13, **dadurch gekennzeichnet, dass** der Waagenteller (3) höhenverstellbar an der Präzisionswaage gelagert ist.

15. Tablettenprüfsystem umfassend eine Präzisionswaage gemäss einem der Ansprüche 13 oder 14.

## Claims

1. Method for determining a weighing and repetition accuracy of a precision balance (1), in particular a precision balance in a tablet testing system, which has a balance plate (3) for placing a test object, **characterized in that** the method comprises the following steps:
(a) provision of a positioning device (7) for the automated positioning of a reference weight (11) on the balance plate (3), a drive unit for movably driving the positioning device (7) relative to the balance plate (3), and a determination unit,
(b) recording of a tare measurement value while the balance plate (3) is empty in order to tare the precision balance (1),
(c) automated positioning of a reference weight (11) on the balance plate (3) by means of the positioning device (7),
(d) recording of a first weighing measurement value for the reference weight (11) by means of the determination unit,
(e) automated removal of the reference weight (11) from the balance plate (3) and subsequent repositioning of the same reference weight (11) on the balance plate (3) by means of the positioning device (7),
(f) recording of a second weighing measurement value for the reference weight (11) by means of the determination unit, and
(g) determination of the weighing and repetition accuracy of the precision balance (1) from the recorded weighing measurement values by the determination unit.

2. Method for determining a weighing and repetition accuracy of a precision balance according to claim 1, wherein steps (e) and (f) are repeated several times in order to record a plurality of weighing measurement values.

3. Method for determining a weighing and repetition accuracy of a precision balance according to either of claims 1 or 2, wherein, at least before the recording of a tare measurement value, a cover is arranged over the balance plate (3) and the positioning device (7), which cover protects the balance plate (3) from environmental influences.

4. Method for determining a weighing and repetition accuracy of a precision balance according to any of claims 1 to 3, wherein the balance plate (3) is cleaned by a cleaning unit (10) driven by the drive unit before the recording of the tare measurement value and/or before the positioning of the reference weight (11).

5. System for determining a weighing and repetition accuracy of a precision balance (1), in particular a precision balance in a tablet testing system, which has a balance plate (3) for placing a test object, **characterized in that** the system comprises:
- a positioning device (7) for the automated positioning of a reference weight (11) on the balance plate (3),
- a drive unit for driving the positioning device (7) relative to the balance plate (3), and
- a determination unit which is coupled to the balance plate (3) for recording weighing measurement values and determines the weighing and repetition accuracy of the precision balance (1).

6. System for determining a weighing and repetition accuracy of a precision balance according to claim 5, **characterized in that** a removable cover is provided which is arranged at least over the balance plate (3) and the positioning device (7) and protects the balance plate (3) from environmental influences.

7. System for determining a weighing and repetition accuracy of a precision balance according to claim 5 or 6, **characterized in that** the positioning device (7), the drive unit and/or the determination unit are provided by structural modules of the precision balance or the tablet testing system and/or are coupled to structural modules of the precision balance or the tablet testing system, for example a drive module, a control module and/or a weighing module of the precision balance and/or a tablet testing system.

8. System for determining a weighing and repetition accuracy of a precision balance according to any of claims 5 to 7, **characterized in that** the positioning device (7) is designed as an at least predominantly vertically movable gripper for the automated gripping and release of the reference weight (11).

9. System for determining a weighing and repetition accuracy of a precision balance according to any of claims 5 to 7, **characterized in that** the positioning device (7) is designed as a horizontally movable receptacle (9, 9') for the automated displacement of the reference weight (11).

10. System for determining a weighing and repetition accuracy of a precision balance according to claim 9, **characterized in that** the receptacle (9, 9') is designed in a sleeve-like manner for loosely receiving a reference weight (11), and a flat platform (5) is provided which forms a plane with the balance plate (3) and at least partially encloses the balance plate (3), the receptacle (9, 9') being arranged to be horizontally displaceable in such a way that it can be moved from the platform (5) over the balance plate (3) and from the balance plate (3) over the platform (5).

11. System for determining a weighing and repetition accuracy of a precision balance according to any of claims 9 to 10, **characterized in that** a second receptacle (9') is arranged on the drive unit in such a way that the second receptacle (9') can be moved from the platform (5) over the balance plate (3), the second receptacle preferably being identical to the receptacle (9) for the reference weight (11).

12. System for determining a weighing and repetition accuracy of a precision balance according to any of claims 9 to 11, **characterized in that** the sleeve-shaped receptacle has a round diameter.

13. Precision balance, in particular for a tablet testing system, with a balance plate (3) for placing a test object, which balance comprises a system for determining a weighing and repetition accuracy according to any of claims 5 to 12.

14. Precision balance according to claim 13, **characterized in that** the balance plate (3) is mounted on the precision balance in a height-adjustable manner.

15. Tablet testing system comprising a precision balance according to either of claims 13 or 14.

## Revendications

1. Procédé permettant de déterminer une précision de pesage et de répétabilité d'une balance de précision (1), en particulier d'une balance de précision dans un système d'essai de comprimés, qui présente un plateau de balance (3) permettant de poser un objet à mesurer, **caractérisé en ce que** le procédé comprend les étapes suivantes :
(a) fourniture d'un dispositif de positionnement (7) permettant de positionner de manière automatisée un poids de référence (11) sur le plateau de balance (3), d'une unité d'entraînement permettant d'entraîner le dispositif de positionnement (7) de manière mobile par rapport au plateau de balance (3), et d'une unité de détermination,
(b) enregistrement d'une valeur de mesure de tarage pendant que le plateau de balance (3) est vide pour le tarage de la balance de précision (1),
(c) positionnement automatisé d'un poids de référence (11) sur le plateau de balance (3) au moyen du dispositif de positionnement (7),
(d) enregistrement d'une première valeur de mesure de pesage pour le poids de référence (11) au moyen de l'unité de détermination,
(e) fait de retirer de manière automatisée le poids de référence (11) du plateau de balance (3), puis de repositionner le même poids de référence (11) sur le plateau de balance (3) au moyen du dispositif de positionnement (7),
(f) enregistrement d'une seconde valeur de mesure de pesage pour le poids de référence (11) au moyen de l'unité de détermination, et
(g) détermination de la précision de pesage et de répétabilité de la balance de précision (1) à partir des valeurs de mesure de pesage enregistrées par l'unité de détermination.

2. Procédé permettant de déterminer une précision de pesage et de répétabilité d'une balance de précision selon la revendication 1, dans lequel les étapes (e) et (f) sont répétées plusieurs fois afin d'enregistrer plusieurs valeurs de mesure de pesage.

3. Procédé permettant de déterminer une précision de pesage et de répétabilité d'une balance de précision selon l'une des revendications 1 ou 2, dans lequel, au moins avant l'enregistrement d'une valeur de mesure de tarage, un élément de recouvrement est disposé au-dessus du plateau de balance (3) et du dispositif de positionnement (7), lequel élément de recouvrement protège le plateau de balance (3) des influences environnementales.

4. Procédé permettant de déterminer une précision de pesage et de répétabilité d'une balance de précision selon l'une des revendications 1 à 3, dans lequel le plateau de balance (3) est nettoyé par une unité de nettoyage (10) entraînée par l'unité d'entraînement avant l'enregistrement de la valeur de mesure de tarage et/ou avant le positionnement du poids de référence (11).

5. Système permettant de déterminer une précision de pesage et de répétabilité d'une balance de précision (1), en particulier d'une balance de précision dans un système d'essai de comprimés, qui présente un plateau de balance (3) permettant de poser un objet à mesurer, **caractérisé en ce que** le système comprend :
- un dispositif de positionnement (7) pour le positionnement automatisé d'un poids de référence (11) sur le plateau de balance (3),
- une unité d'entraînement pour l'entraînement du dispositif de positionnement (7) par rapport au plateau de balance (3), et
- une unité de détermination qui est couplée au plateau de balance (3) pour l'enregistrement de mesures de pesage et qui détermine la précision de pesage et de répétabilité de la balance de précision (1).

6. Système permettant de déterminer une précision de pesage et de répétabilité d'une balance de précision selon la revendication 5,
**caractérisé en ce qu'**un élément de recouvrement amovible est prévu, lequel est disposé au moins au-dessus du plateau de balance (3) et du dispositif de positionnement (7) et protège le plateau de balance (3) des influences environnementales.

7. Système permettant de déterminer une précision de pesage et de répétabilité d'une balance de précision selon la revendication 5 ou 6, **caractérisé en ce que** le dispositif de positionnement (7), l'unité d'entraînement et/ou l'unité de détermination sont donnés par des modules structurels de la balance de précision ou du système d'essai de comprimés et/ou sont couplés à des modules structurels de la balance de précision ou du système d'essai de comprimés, par exemple à un module d'entraînement, à un module de commande et/ou à un module de pesage de la balance de précision et/ou d'un système d'essai de comprimés.

8. Système permettant de déterminer une précision de pesage et de répétabilité d'une balance de précision selon l'une des revendications 5 à 7,
**caractérisé en ce que** le dispositif de positionnement (7) est réalisé sous forme de préhenseur mobile au moins principalement verticalement pour la saisie et la libération automatisées du poids de référence (11).

9. Système permettant de déterminer une précision de pesage et de répétabilité d'une balance de précision selon l'une des revendications 5 à 7, **caractérisé en ce que** le dispositif de positionnement (7) est réalisé sous forme de logement (9, 9') mobile horizontalement pour le coulissement automatisé du poids de référence (11).

10. Système permettant de déterminer une précision de pesage et de répétabilité d'une balance de précision selon la revendication 9, **caractérisé en ce que** le logement (9, 9') est réalisé sous forme de douille pour recevoir librement un poids de référence (11) et une plate-forme (5) plane est prévue, laquelle forme un plan avec le plateau de balance (3) et entoure au moins partiellement le plateau de balance (3), dans lequel le logement (9, 9') est disposé de manière à pouvoir être coulissé horizontalement de telle sorte qu'il est mobile de la plate-forme (5) à au-dessus du plateau de balance (3) et du plateau de balance (3) à au-dessus de la plate-forme (5).

11. Système permettant de déterminer une précision de pesage et de répétabilité d'une balance de précision selon l'une des revendications 9 à 10, **caractérisé en ce qu'**un second logement (9') est disposé sur l'unité d'entraînement de telle sorte que le second logement (9') est mobile de la plate-forme (5) à au-dessus du plateau de balance (3), dans lequel le second logement est de préférence identique au logement (9) pour le poids de référence (11).

12. Système permettant de déterminer une précision de pesage et de répétabilité d'une balance de précision selon l'une des revendications 9 à 11,
**caractérisé en ce que** le logement en forme de douille présente un diamètre rond.

13. Balance de précision, en particulier pour un système d'essai de comprimés, comportant un plateau de balance (3) permettant de poser un objet à mesurer et comprenant un système permettant de déterminer une précision de pesage et de répétabilité selon l'une des revendications 5 à 12.

14. Balance de précision selon la revendication 13, **caractérisée en ce que** le plateau de balance (3) est monté sur la balance de précision de manière à être réglable en hauteur.

15. Système d'essai de comprimés comprenant une balance de précision selon l'une des revendications 13 ou 14.
